(19) **Europäisches Patentamt European Patent Office Office européen des brevets**

(11) **EP 4 401 618 B1**

(12) **EUROPEAN PATENT SPECIFICATION**

(45) Date of publication and mention of the grant of the patent:
**25.12.2024 Bulletin 2024/52**

(21) Application number: **22790242.6**

(22) Date of filing: **14.09.2022**

(51) International Patent Classification (IPC):
***A61B 5/00*** *(2006.01)*

(52) Cooperative Patent Classification (CPC):
**A61B 5/443; A61B 5/7225; A61B 5/7228**

(86) International application number:
**PCT/EP2022/075468**

(87) International publication number:
**WO 2023/041549 (23.03.2023 Gazette 2023/12)**

(54) **APPARATUS AND METHOD FOR MEASURING SKIN CHARACTERISTIC**

VORRICHTUNG UND VERFAHREN ZUR MESSUNG DER HAUTCHARAKTERISTIK

APPAREIL ET PROCÉDÉ DE MESURE DE CARACTÉRISTIQUES DE LA PEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.09.2021 EP 21197191**

(43) Date of publication of application:
**24.07.2024 Bulletin 2024/30**

(73) Proprietor: **Koninklijke Philips N.V.**
**5656 AG Eindhoven (NL)**

(72) Inventors:
• **BOAMFA, Marius Iosif**
**5656 AG Eindhoven (NL)**
• **VERHAGEN, Rieko**
**5656 AG Eindhoven (NL)**
• **VAN ABEELEN, Frank Anton**
**5656 AG Eindhoven (NL)**
• **THUMMA, Kiran Kumar**
**5656 AG Eindhoven (NL)**
• **WARMERDAM, Thomas Petrus Hendricus**
**5656 AG Eindhoven (NL)**
• **VAN KEMPEN, Eric Gerard Marie**
**5656 AG Eindhoven (NL)**
• **BALTAZAR DOS SANTOS, Nuno Filipe**
**5656 AG Eindhoven (NL)**
• **MAZARAKIS, Giorgos**
**5656 AG Eindhoven (NL)**

(74) Representative: **Philips Intellectual Property & Standards**
**High Tech Campus 52**
**5656 AG Eindhoven (NL)**

(56) References cited:
**CN-A- 113 288 416      KR-A- 20210 101 896**
**KR-B1- 102 067 260**

**EP 4 401 618 B1**

**Description**

FIELD OF THE INVENTION

The present invention relates to an apparatus and method thereof, for measuring a skin characteristic. It further relates to the field of personal care devices, more specifically, skin treatment devices.

BACKGROUND OF THE INVENTION

Many skin treatment devices require the determination of a skin characteristic as part of their operation. Skin characteristics used by such devices can include skin tone, skin melanin index, and other parameters that relate to skin color (skin tone/skin pigmentation), skin patterns or skin features (e.g. the presence of hair, moles, scars, acne, etc.). Examples of treatment devices that benefit from determination of skin characteristics include photoepilation devices. Such devices use treatment sources such as laser, LED, flash lamp etc. for epilation. An example is an Intense Pulsed light (IPL) device. Further examples include devices for treating acne and skin lesions.

Intense Pulsed light (IPL) technology is a popular solution for many treatment applications such as, but not limited to, photoepilation, lesion treatment, photo-rejuvenation, in-home personal care, professional personal care and medical settings. IPL photoepilation devices usually apply light across a broad wavelength spectrum to the surface of the skin, targeting the melanin in the hair e.g. in the hair follicles. The hair/follicle absorbs the energy and therefore heats up, leading to an interruption of the hair growth.

A skin characteristic such as a skin tone is typically categorised into groups such as: 'white', 'beige', 'light brown', 'medium brown', 'dark brown' and 'brownish black and darker'. Typically, IPL photoepilation devices are not used with darker skin as the skin will absorb energy in the light pulse rather than the hair or follicle. In that case, if a skin tone of e.g. brownish black and darker, is detected, the device is programmed not to trigger a flash. Typically, "skin tone" is interpreted as relating to the melanin content ("level of brown"). A skin characteristic such as skin color or pigmentation can be used to detect skin erythema.

The determination of skin characteristics enables controlling the energy setting of the device e.g. based on a light/dark skin tone, and for ensuring device operational safety. For example, if an unsuitable skin tone is detected, the device automatically disables flashing. Alternately, a suitable energy level for treatment can be variably selected/adapted based on the skin characteristic.

KR102067260B1 discloses a device for photobiomodulation treatment of the human body by means of infrared light. The device comprises a laser light source for generating the treatment light and an output control module configured to adjust the wavelength or intensity of the treatment light based on the output of a skin tone calculating module. The skin tone calculation module is configured to calculate the skin tone of the user's body by comparing the light reflected by the skin with preset data. The light reflected by the skin is measured by a plurality of light receiving units arranged at different distances from the light source. The preset data may be data representing a range for classifying the optical signals measured according to the distance from the light source. The skin tone is derived from the slope of a curve that represents the measured intensity of the reflected light as a function of the distance of the light receiving unit from the light source.

KR20210101896A discloses an apparatus for improving the PPG pulsation cycle signal quality in a blood glucose meter by using a skin color sensor. The skin color sensor comprises a skin reflection detection unit arranged for measuring the light irradiated towards the skin by means of a white LED and reflected by the skin of the subject to be measured. The skin color sensor further comprises a skin color estimation unit configured for converting the measured light into a color code and determining a skin color type based on the color code. The intensity of the light of the white LED to be generated for the PPG measurement is adjusted in dependence on the determined skin color type.

At least for the above, it is desirable to determine the skin characteristic with sufficient accuracy.

SUMMARY OF THE INVENTION

An apparatus (e.g. a sensor) which measures a skin characteristic may make use of at least one, e.g. two light sources, e.g. LEDs, that operate at two distinct wavelengths (e.g. at 640 nanometres (nm) - wavelength 1 and 870 nm central wavelengths - wavelength 2, respectively). The light from these LEDs is emitted towards the skin and a detector measures the reflected light, resulting in detector signals S 1 and S2 for the two LEDs respectively. Based on the levels of skin reflectivity R for the two wavelengths a skin tone or pigmentation can be computed. The skin tone or pigmentation is a function of the ratio of reflection $\sim \frac{S1}{S2}$ and/or skin reflectivity $\frac{R1}{R2}$ at each wavelength 1, 2.

A problem with such measurements is that the detected signal is influenced by both external factors such as ambient light and temperature, as well as internal reflection losses within the device. The measurements may further be affected by the detector response characteristics (e.g. how it behaves at low intensities). As a result, when said apparatus is used with a personal care device for skin treatment, the device may not effectively classify the skin tone. For example, on one hand, the device may not block certain skin tones when it should, leading to safety concerns. On the other hand, it may erroneously block certain suitable skin tones, preventing a portion of users from using the device.

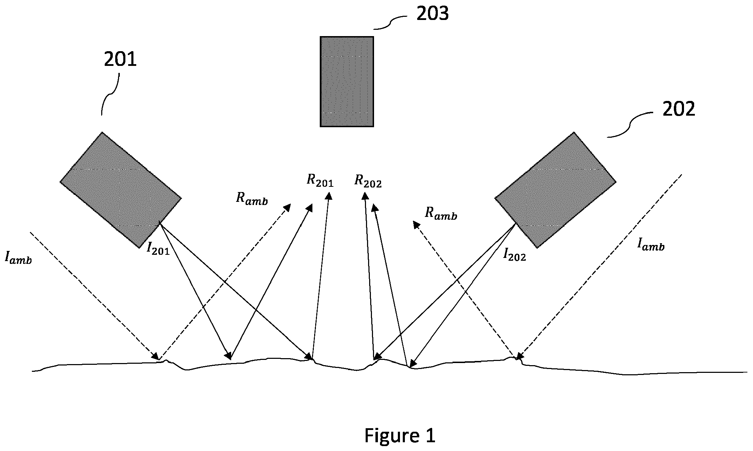

An example is shown in Fig. 1, which shows a prior art set-up for measurement of reflectance signal from skin. Light sources 201 and 202 emit light of central wavelengths $\lambda_1$ and $\lambda_2$, respectively. Exemplary rays $R_{201}$ corresponding to light source 201, and $R_{202}$ corresponding to light source 202, are reflected off the skin and detected by detector 203. In other words, incident ray $I_{201}$ is reflected from the skin as reflected ray $R_{201}$, and incident ray $I_{202}$ is reflected from the skin as reflected ray $R_{202}$. The effective sensing/detection area of detector 203 on the skin corresponds to an area that is illuminated by light sources 20 1 and 202.

At the same time, incident ambient light $I_{amb}$ from the surroundings also gets reflected off the skin towards the same detector 203 as reflectance signals $R_{amb}$. These signals manifest as noise in optical measurements and decrease the signal to noise ratio (S/N) of detection, leading to measurement inaccuracies.

At least for the problems mentioned above, it is an object of the invention to provide an improved method for determination of skin characteristics, e.g. a skin tone, and apparatus thereof, for effective use of the skin treatment device.

In more detail, it is an object of the invention to provide a method and an apparatus thereof which compensates for noise generated by ambient light in the signal measurements, and to improve the S/N (sensitivity) of detection of the skin characteristic.

It is another object of the invention to provide a signal measurement method which takes into account source and detector (and associated circuitry) behavior at different incident intensity levels.

It is yet another object of the invention to provide a signal measurement method which compensates for reflections of the incident light within the device giving rise to spurious signals.

It is yet another object of the invention to provide a signal measurement method and an apparatus thereof, which compensates for signal deviation generated by temperature of the environment.

The description herein is intended to overcome at least one of the problems mentioned above.

According to an aspect of the invention, an apparatus for measuring a skin characteristic is provided. The apparatus comprises a processor configured to receive, over a time period T, a first response indicating intensities of light of a first wavelength $\lambda_1$ reflected from skin, and a second response indicating intensities of light of a second wavelength $\lambda_2$ reflected from the skin, receive a first indication of a total intensity of light corresponding to the first response and a second indication of a total intensity of light corresponding to the second response, which are emitted to the skin over the time period T, determine a first variation curve (10) with the first response as a function of the first indication and a second variation curve (11) with the second response as a function of the second indication, determine a portion (10', 11') of each variation curve, along which portion (10', 11') the variation is substantially linear, calculate slopes at at least one point in said portion of the variation curves, and determine a value of the skin characteristic from a ratio of the slopes. The indication and response may include values of voltage, current or intensity. For example, in one aspect, the first/response and first/second indication comprise voltage or current values which are indicators of reflected and emitted intensities, respectively. In another aspect, these are values/levels of reflected and emitted intensities.

According to an aspect of the invention, the apparatus further comprises a signal generator configured to modulate an intensity of the emitted light by the first light source at a first frequency f1 and the second light source at a second frequency f2 across a plurality of intensities during the time period T. In cases where the emitted intensities are modulated, the processor is configured to receive over the time period T, a first modulated response and a second modulated response.

According to an aspect of the invention, the processor is further configured to adjust the first response and the second response based on an intensity of a part of the total emitted light reflected internally within the optical sensor wherein the intensity of said part is determined based on total light intensities emitted over the time period T and associated with said portion of the variation curve.

According to an aspect of the invention, the apparatus further comprises a temperature sensor, and wherein the processor is further configured to adjust the first response and/or the second response based on a measurement of the temperature sensor.

According to an aspect of the invention, the apparatus further comprises a memory to store a calibration correction for the first response and/or the second response, and wherein the processor is further configured to calibrate a reflection ratio S or a skin characteristic based on the calibration correction.

According to an aspect of the invention, a personal care device is provided, the device comprising the apparatus at least as described above, and/or wherein the apparatus is included in an attachment of the personal care device.

According to an aspect of the invention, a computer-implemented method for determining a skin characteristic is provided. The method comprises receiving, over a time period T, a first response indicating intensities of light of a first

wavelength $\lambda_1$ reflected from skin, and a second response indicating intensities of light of a second wavelength $\lambda_2$ reflected from the skin, receiving a first indication of a total intensity of light corresponding to the first response and a second indication of a total intensity of light corresponding to the second response, which are emitted to the skin over the time period T, determining a first variation curve with the first response as a function of the first indication and a second variation curve with the second response as a function of the second indication, determining a portion of each variation curve, along which portion the variation is substantially linear, calculating (305) slopes at at least one point in said portion of the variation curves, and determining (306) a value of the skin characteristic from a ratio of the slopes.

According to an aspect of the invention, a computer program product or a computer-readable storage medium comprising instructions which, when the program is executed by a computer, cause the computer to carry out the steps of the method. These and other aspects, and further advantages, will be apparent from and elucidated with reference to the embodiment(s) described herein.

BRIEF DESCRIPTION OF THE DRAWINGS

Fig. 1 shows an arrangement according to a prior art method.

Figs. 2a, 2b show a signal detection method according to an exemplary aspect of the present invention.

Fig. 3 shows a signal detection method according to another exemplary embodiment of the present invention.

Fig. 4 shows a first arrangement for measuring skin characteristics according to an embodiment of an invention.

Fig. 5a shows a second arrangement for measuring skin characteristics according to an embodiment of an invention.

Fig. 5b shows a third arrangement for measuring skin characteristics according to an embodiment of an invention.

Fig. 6 shows a method for measuring skin characteristics according to an embodiment of the invention.

Fig. 7 shows a method for measuring skin characteristics according to another embodiment of the invention.

Fig. 8 shows an illustration of apparatus 200 according to an embodiment of the invention.

Fig. 9 shows an illustration of apparatus 200 according to another embodiment of the invention.

DETAILED DESCRIPTION OF THE EMBODIMENTS

The matters exemplified in this description are provided to assist in a comprehensive understanding of various exemplary embodiments of the present invention disclosed with reference to the accompanying figures.

Accordingly, those of ordinary skill in the art will recognize that various changes and modifications of the exemplary figures/embodiments described herein can be made without departing from the scope of the claimed invention. In particular, combinations of specific features of various aspects of the invention may be made. An aspect or embodiment of the invention may be further advantageously enhanced by adding a feature that was described in relation to another aspect or embodiment of the invention.

Further, the functionality associated with any particular means may be centralized or distributed, whether locally or remotely. A computer program may be stored/distributed on a suitable medium, such as an optical storage medium or a solid-state medium supplied together with or as part of other hardware, but may also be distributed in other forms, such as via the Internet or other wired or wireless telecommunication systems. It may be advantageous to set forth that the terms "include" and "comprise," as well as derivatives thereof, mean inclusion without limitation. In addition, reference to an element by the indefinite article "a" or "an" does not exclude the possibility that more than one of the element is present, unless the context clearly requires that there be one and only one of the elements. The indefinite article "a" or "an" thus usually means "at least one".

The expression "at least one of A, B and C" means "A, B, and/or C", and that it suffices if e.g. only B is present. Any reference signs in the claims should not be construed as limiting the scope.

The term "reflected" used herein encompasses light reflected from the skin surface as well as that reflected after being scattered from the skin surface and/or interior.

The term "skin" used herein encompasses skin of a user while using the personal care device and a reference material mimicking skin.

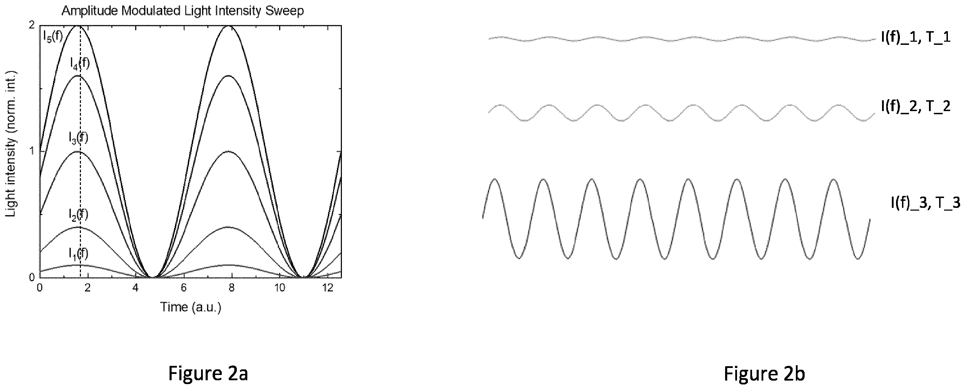

Figs. 2a, 2b show a signal detection method according to an exemplary aspect of the present invention, and corresponds to amplitude (or intensity, which is expressed as a value proportional to square of amplitude) modulation. In principle, the method may be extended to any type of signal modulation as known in art (phase, polarization etc.). The method achieves a better reduction of noise due to ambient light.

Amplitude or intensity modulation techniques are those wherein an amplitude or intensity of a carrier signal is modified in time using a modulating signal having a modulation frequency f. The modulation yields a signal with a modulated amplitude or intensity, which is at least based on the modulation frequency f. Fig. 2a shows a general representation of amplitude/intensity modulation of a plurality of light waves [superposed] in time.

For example, a light source as used in the present invention can be arranged to emit signals of varying intensities, $I\_1$ to $I\_i$ (i=5, in Fig. 2a). Preferably, the intensities emitted by the light source are varied stepwise, as shown in Fig. 2b, in + or - directions, for a certain duration or period T (called an intensity sweep). These intensities are modulated using a modulating frequency f. The peak intensities of the modulated signals are shown to correspond to $I(f)\_1$, $I(f)\_2$, ...., $I(f)\_i$, i> 1, spanning an intensity range from low to high intensities.

In Fig. 2b, at times $T\_1$, $T\_2$ and $T\_3$, the light source emits light with peak intensity $I(f)\_1$, $I(f)\_2$ and $I(f)\_3$, respectively. Period or duration $T=T\_1+T\_2+T\_3$, is otherwise called a duration of a measurement sweep.

Only light which is emitted by the light source is modulated and therefore linked to the modulating frequency f component. The light is reflected by the skin surface and detected by detector 203. It can be demodulated and/or filtered out using suitable detection circuitry such as band pass filters, lock-in amplifiers or other signal demodulators. The ambient light remains unmodulated or may be modulated at a different frequency (e.g. frequency of the electricity grid (50 or 60Hz) and harmonics thereof), and hence without association to the modulating frequency f. The detector 203 can be configured to detect and process signals (or transmit them for signal processing by control circuitry) which are associated with modulation frequency f, and disregard other detected signals, in this case, those which contribute to noise in a measurement.

Although the arrangement shows two light sources, from the above discussion, it is clear to the skilled person that it is sufficient to modulate intensities of a single light source to filter noise from ambient light. The use of two light sources is however desirable, as such measurement makes the method less prone to noise and decreases the number of additional steps (mathematical corrections, further measurements) otherwise required to compensate for this noise.

In order to accommodate reflectivities of different types of skin, in an embodiment, it is desirable to obtain skin characteristic information using a larger range of emitted intensities I(f), and consequently reflected intensities I'(f), which spans from low to high intensity values in an operating range of the light source and the detector. For example, the darker skin tones reflect too little light, and lighter skin tones too much.

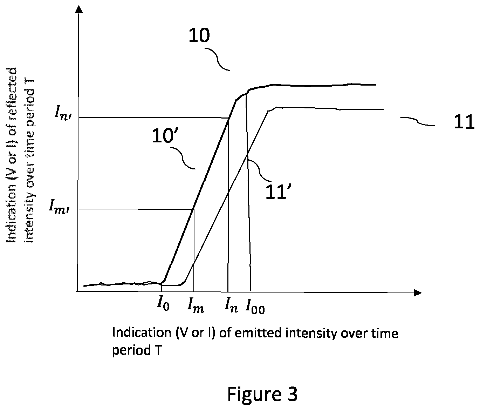

Fig. 3 shows a signal detection method according to another exemplary embodiment of the present invention.

In Fig. 3, values of a suitable indication, e.g. driving voltages or currents representing emitted intensities of light source 201 and light source 202 during time period T are plotted along the x axis. These intensities may be modulated as explained above if ambient light reduction is desired. As an example, $I\_m$ and $I\_n$ correspond to two intensity values emitted by source 201.

Values of a suitable response (e.g. voltage or current values) parameter measured by the light detector 203 during the same time period T (for each emitted intensity), which indicate the reflected intensities, are plotted along the y axis. If the source intensities are modulated, the reflected intensities are also modulated. These signals are then demodulated by the detector 203 or associated signal processing circuitry, as mentioned above. $I\_m'$ and $I\_n'$ correspond to two intensity values of light reflected (as mentioned, directly from the skin surface or scattered internally and reflected) from the skin. $I\_m'$ and $I\_n'$ are reflected intensities corresponding to $I\_m$ and $I\_n$, respectively.

Curves 10 and 11 represent the responsivity of detector 203 over time period T. A detector such as a photodiode outputs a current value (or any other parameter) based on the input light (e.g. photon count) it detects at a certain wavelength or a range of wavelengths, and the ratio of the output current to input current at the respective wavelength gives a measure of responsivity of the detector. The output current/voltage varies proportionally with the input current/power and gives a measure of the photon count registered by the detector. It is understood that the shape of the response curve may deviate from the exemplary variation shown in Fig. 3.

Curves 10 and 11 shows the response of detector 203 corresponding to reflected intensities of wavelengths $\lambda_1$ by source 201 and $\lambda_2$ by source 202, with respect to said indication of emitted intensities from the respective source. Assuming an LED to be a light source and a photodiode to be a light detector, the curve could e.g. depict a voltage-voltage relationship (voltage input to the LED driver - voltage measured at the output of the photodiode).

Irrespective of whether intensities of the emitted light (and therefore the reflected light) are modulated, when the emitted intensity of either light source 201 or 202 (or the driving voltage or current to the source) is below a certain minimum threshold, the intensity of the reflected light (and therefore the response registered at detector 203) is correspondingly weak. In such cases, detector 203 and/or associated circuitry show a non-linear response characteristic, which typically results in noise in the measurement. This is termed as noise regime of a detector.

Above the minimum threshold intensity level/value I_0 (or the corresponding indication), the output (intensity/current/voltage) registered at detector 203 shows a substantially linear relationship with the input (intensity/current/voltage). In other words, the response variation is substantially linear. This is termed as the linear regime of a detector.

Correspondingly, detector 203 outputs a substantially linear response or a linear variation of the reflected intensity or the current/voltage with respect to the source intensity/current/voltage. Detector 203 can be configured to exhibit a linear response between a lower threshold I_0 and an upper threshold I_00. This linear regime is shown by substantially linear portions 10' and 11' of the detector response curves 10 and 11, respectively. It is known to the skilled person that in practice, linear portions 10' and 11' of the response curves 10 and 11 may still have a degree of curvature.

Above the upper threshold I_00, said variation may not show a linear relationship, in this case, due to saturation of the detector. This means that the reflected intensity detected by detector 203 (or current or voltage) does not vary anymore, or minimally varies, with a corresponding variation in the emitted intensity (or current or voltage). This is termed as the detector saturation regime in art.

Thresholds 1_0 and I_00 may vary depending on the physical and electrical characteristics of the light source, light detector and associated circuitry.

The method and device according to the present invention takes into account the behavior characteristics of the sources, the detector and associated circuitry to determine skin characteristics of the user of the device by determining and processing information in the linear response regime of the detector.

The reflected intensity detected by the detector 203 is also affected by the reflectivity of the skin. For example, some skin tones reflect too little light, and others too much. If the skin reflectivity is low, the reflected intensities detected by detector 203 are weak, which could give rise to high noise relative to a low signal. In this case, obtaining signal information from the linear regime of the response curve provides optimum detection results.

If the skin reflectivity is high, the reflected intensities detected by detector 203 are very strong, which could give rise to detector saturation even at relatively low emitted intensities. In this case like above, obtaining signal information from the linear regime of the response curve provides optimum detection results.

It is desirable to modulate the intensities emitted from light sources 201 and/or 202 to achieve reduction in ambient noise in the reflectance signal measurements.

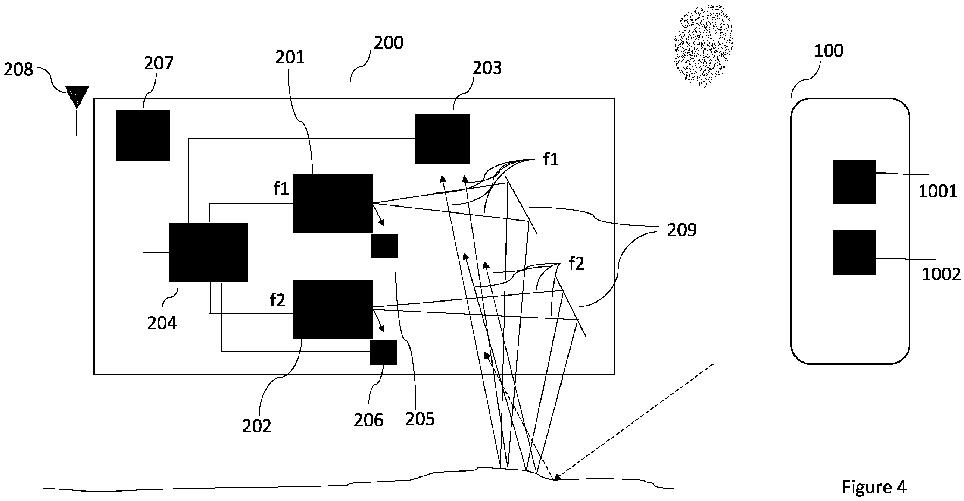

Fig. 4 shows a first arrangement for measuring skin characteristics according to an embodiment of an invention.

According to the embodiment, an apparatus 100 for measuring skin characteristic as mentioned above is part of a wireless communication network, e.g. may be located in a cloud computing/distributed server or in another processing device (e.g. a smartphone, a personal computer). Apparatus 100 comprises a processor 1001 which is configured to receive, over a time period T, a first (detector) response comprising and/or indicating reflected intensities (from skin) of light of a first wavelength $\lambda_1$. The intensities may be modulated at a frequency f1. It further receives, over the period T, a second (detector) response comprising and/or indicating reflected intensities (from skin) of light of a second wavelength $\lambda_2$. The intensities may also be modulated at a frequency f2. The first and second response comprises reflected intensities and/or response parameters such as current or voltage values, which provide an indication of said reflected intensities. Processor 1001 may be a general processor which is e.g. implemented in a user terminal such as a smartphone.

The first response and the second response are received from a client device 200. The client device 200 may be the personal care (e.g. IPL) device for treatment of skin, or an optical sensor (or any other type of sensor) in the personal care device. In case, device 200 is the personal care device, the optical sensor may be comprised therein as one of its components, e.g., a device attachment. Transceiver 207 is configured to transmit and receive wireless signals over the wireless communication network via antenna 208.

The client device 200 further comprises the light sources 201 and 202, which are configured to emit light of wavelengths $\lambda_1$ and $\lambda_2$, respectively, when device 200 is operated by a user by positioning device 200 on the skin. The light sources 201 and 202 (e.g. LEDs, laser diodes, surface emitting lasers) are configured to function independently, with respect to driving voltage/current and with respect to the time domain (can be turned on simultaneously or sequentially).

Intensities I_201_1, 1_201_2, ...., I_201_i, i> 1 are emitted from light source 201 towards the skin over a time period T. These intensities are reflected from the skin, towards detector 203. Detector 203 detects the reflected intensities over period T (the first response) and transmits the obtained reflected intensities to the signal processing circuitry 204. The intensities may be transmitted in real time, or after period T. In an embodiment, a dummy or reference material mimicking skin is used for these intensity measurements.

In an embodiment, the signal processing circuitry may further comprise a signal generator which is configured to apply voltage or current to source 201 to modulate the emitted intensities, and hence the reflected intensities, during period T at a modulating frequency f1.

Similarly, intensities I_202_1, I_202_2, ... I_202_i, i> 1 are emitted from light source 202 towards the skin over the same time period T. These intensities are also reflected from the skin, towards detector 203. Detector 203 detects the reflected intensities over period T (the second response) and transmits the obtained reflected intensities to the signal processing circuitry 204. As above, the intensities may be transmitted in real time, or after period T. As mentioned, in an embodiment, a dummy or reference material mimicking skin is used for these intensity measurements.

In Fig. 4, these intensities are directed to the skin via guiding elements 209. Mirrors 209 are shown for this purpose. It is clear to the skilled person that depending on the geometrical arrangement of the components (e.g. source 201, 202, detector 203), these guiding elements may be omitted or be replaced with other suitable optical elements. The geometry of the guiding elements may be so chosen to maximize the geometrical overlap of the light emitted by the light sources 201 and 202.

In an embodiment, light source 201 is configured to emit light of wavelength in the visible region of the electromagnetic spectrum, and light source 202, in the infrared region. At these wavelengths, melanin has a relatively high absorption coefficient, so that the light is effectively absorbed by the pigment. Hence, the reflectance measurements and consequently the calculation of the skin tone are optimized. In an embodiment, light sources 201 and 202 may have peak emission wavelengths around 660 nm (visible, red) and 880 nm (Infrared), respectively. Alternately or additionally, light source 201 may have peak emission wavelength around 550 nm (visible, green). Light source 201 can be tailored to measure different skin properties or characteristics by choosing the respective wavelength. A tunable light source or a plurality of light sources each operating at the desired wavelength(s) may be used.

Detector 203 may be a photodiode, e.g. a silicon photodiode, having a detection sensitivity between 400 nm to 1100 nm, and therefore shows a variation in electrical behavior as a function of the intensity of incident light for these wavelengths.

Although a single detector 203 is shown used to detect both the first response and the second response, separate detectors may also be configured to detect the respective response.

In an embodiment, the signal processing circuitry may further comprise a signal generator which is configured to apply voltage or current to source 202 to modulate the emitted intensities, and hence the reflected intensities, during period T at a modulating frequency f2.

As mentioned, the noise generating ambient light remains unmodulated, and therefore can be distinguished from the reflected signals

In an embodiment, the modulating frequencies f1 and f2 may be the same or different.

In yet another embodiment, the intensities of each source 201 and 202 may be modulated, either sequentially or simultaneously, during period T or unmodulated.

Modulating light signals with different frequencies f1, f2 simultaneously allows measuring both colors or wavelengths at the same time with a single detector and without further optical filters.

Processor 1001 further receives total intensities of light emitted over time period T by each light source 201 and 202 towards the skin (the incident light). The term total intensity herein refers to the intensity emitted by a light source. The response indicates an intensity which is reflected from the skin, after at least a part of this total intensity is incident on it. As will be clear from below, a part of this total intensity may be internally reflected in the device. These (total) intensities may emitted in a stepwise manner from a lower to an upper range.

Alternately or additionally, processor 1001 may receive an indication such as a voltage or current for driving the light sources 201/202, the values of which indicate the intensities emitted by the sources. The response (voltage, current) in this case provides an indication of the reflected intensities.

The first response hence is associated with an intensity of light which is reflected from the skin, from a first total intensity of the light which is incident on the skin over period T. Similarly, the second response is associated with an intensity of light which is reflected from the skin, from a second total intensity of the light which is incident on the skin over period T.

The client device 200 is configured to transmit each intensity value $I\_201\_1$, $I\_201\_2$, ...., $I\_201\_i$, $i > 1$ and $I\_202\_1$, $I\_202\_2$, ...., $I\_202\_i$, $i > 1$, emitted by the light sources during time period T and/or an indication, e.g. voltage values $V\_201\_1$, $V\_201\_2$, ...., $V\_201\_i$, $i > 1$ and $V\_202\_1$, $V\_202\_2$, ...., $V\_202\_i$, $i > 1$ associated thereto and correspondingly, each value of the first response and second response to the processor 1001 of apparatus 100.

The client device 200 may further comprise further light detectors 205, 206 configured to measure the intensity values $1\_201\_1$, $I\_201\_2$, ...., $I\_201\_i$, $i > 1$ and $I\_202\_1$, $I\_202\_2$, ...., $I\_202\_i$, $i > 1$, respectively, as these intensities are emitted by sources 201 and 202. In Fig. 4, detectors 205 and 206 are shown connected to signal processing circuitry 204, which further transmits these values to processor 1001 via the transceiver 207 and antenna 208. Any alternate arrangement to transmit the emitted intensity values to processor 1001 may also be used. It is also possible to use a single further detector to detect both wavelengths. The detector is preferably disposed near the light source, in order to capture the total intensity without losses to environment. Processor 200 is further configured to determine a first variation 10 of the first response (comprising the reflected intensities) with the first total intensity (comprising emitted intensities incident on skin). The variation is obtained as a curve, as discussed above. It is further configured to determine a second variation 11 of the second response (comprising the reflected intensities) with the second total intensity (comprising emitted intensities incident on skin).

Processor 1001 further determines a portion 10', 11' of each variation curve, which corresponds to the aforementioned linear regime. Along portions 10', 11', the first variation curve 10 and the second variation curve 11 take a substantially linear shape. A certain degree of curvature may exist despite the linear relationship. It then determines a value of the skin characteristic by calculating a ratio of a slope at at least one point along portion 10', $\alpha_1$, and a slope at at least one point along portion 11', $\alpha_2$.

The ratio of the slopes $\dfrac{\alpha_1}{\alpha_2}$ is proportional to the ratio of the reflected intensities S 1 and S2 corresponding to the two wavelengths (reflection ratio S~S1/S2), dependent on the skin reflectivity at these wavelengths (explained below). The reflection ratio S can be mapped to the skin characteristic.

The advantage of using the linear portion of the curve is that one excludes those regions that are not useful/sufficiently accurate for skin characteristic measurement and uses only the optimal part of the response curve.

The mapping of the skin characteristic by extraction of the slope ensures that a plurality of intensity points, and hence a differential absorbance/reflectance is taken into account for calculation of the skin characteristic. Further, since a linear slope is calculated, the method ensures that data processing is performed using measured data points in said linear portion of the curve, and not e.g. in the non-linear or saturation regime. Apparatus 100 further comprises a memory 1002 to store at least calibration data associated with the device 200. These data may include corrections for temperature, internal reflection, skin characteristics mapping, information on the linear regime of the optical sensor etc.

Apparatus 100 may transmit the measured skin characteristic and/or calibration data to client device 200. This may be done real-time, as client device 200 is used by the user.

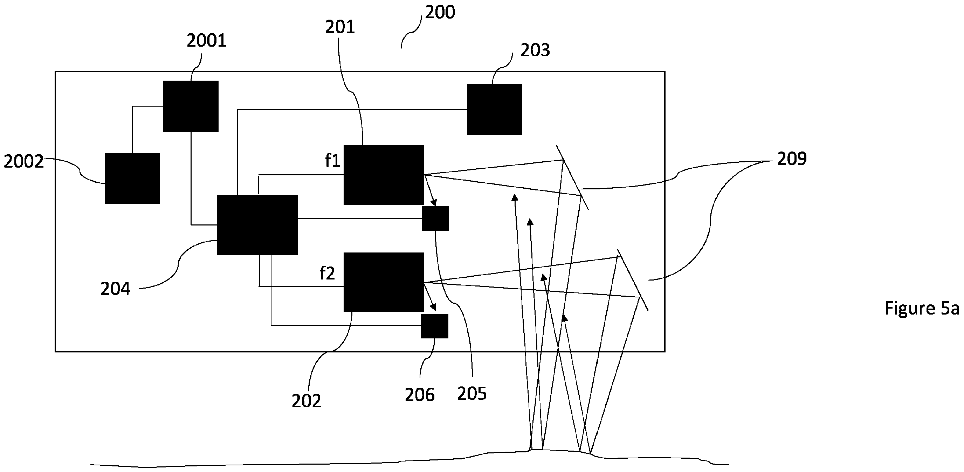

In an embodiment, apparatus 100 may be used to measure the skin characteristic using reference/dummy skin data which are prior collected, for example, by client device 200 or another suitable device. This is particularly advantageous in a factory setting while manufacturing device 200. The measured skin characteristic and/or calibration data may be transmitted to the client device 200 to be stored therein. The information can be processed by the client device 200 as reference data, to further optimize a skin characteristic which it measures real-time when used by the user. If information about the linear regime is then stored in the client device 200, a further determination of the variation curves 10, 11 and the linear regime may be omitted by the client device 200 in real-time measurements. This makes determination of the skin characteristic faster while using the client/personal care device. Fig. 5a shows a second arrangement for measuring skin characteristics according to an embodiment of an invention.

In this embodiment, processor 2001 and memory 2002 are comprised within the client device 200. The signal processing circuitry 204 described in the embodiment of Fig. 4 may be disposed separately from processor 2001 or be integrated therewith. Processor 2001 is configured to perform steps described in connection with processor 1001. Similar to Fig. 4, processor 2001 may further optimize a skin characteristic, which is measured by the personal care/client device 200 when used by the user, based on prior measured skin characteristic and/or calibration data (as reference measurements). If memory 2002 includes predetermined information on the linear regime of the optical sensor of the personal care/client device 200, the personal care/client device may omit a further determination of the variation curves and the linear portion when the device is used by the user.

The remaining features of this embodiment, and their functions are the same as embodiment of Fig. 4 and shall not be repeated for sake of conciseness.

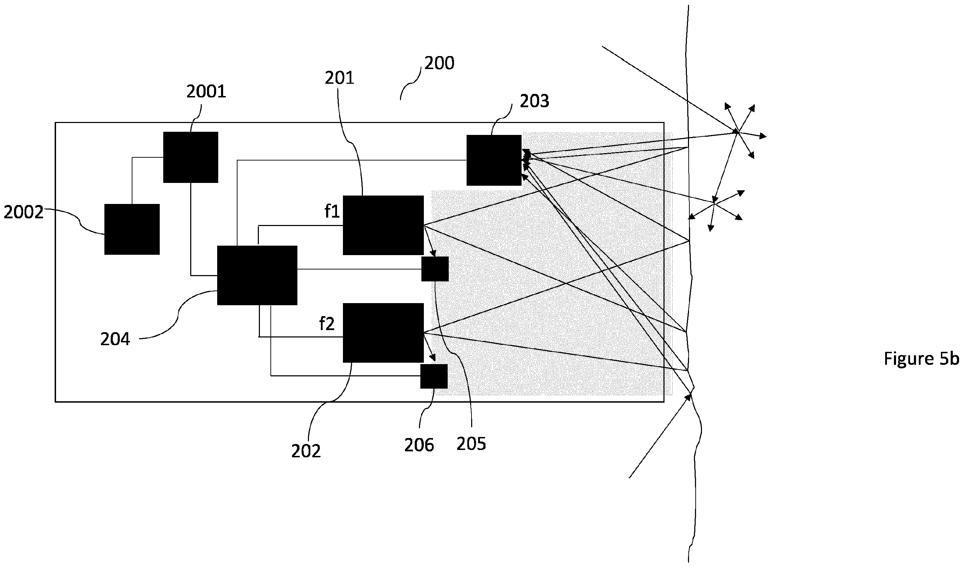

Fig. 5b shows a third arrangement for measuring skin characteristics according to an embodiment of an invention.

It shows an implementation without guiding elements 209 shown in Figs. 4 and 5a. As mentioned above, elements 209 may be omitted in either embodiment of Fig. 4 or Fig. 5a. The figure further shows how incident light is scattered from within the skin and reflected towards detector 203.

The remaining features of this embodiment, and their functions are the same as embodiment of Fig. 5a and shall not be repeated for sake of conciseness.

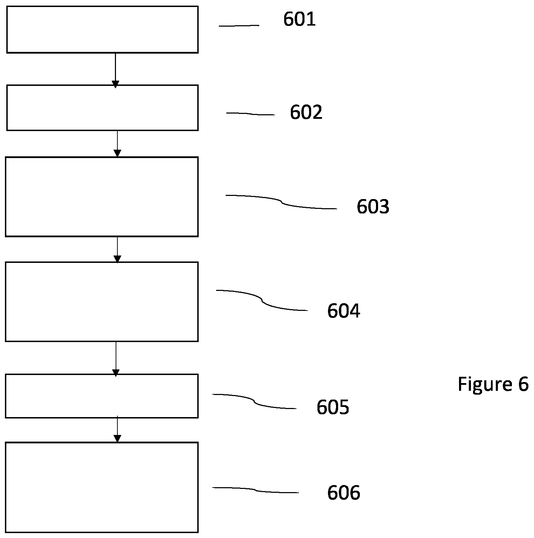

Fig. 6 shows a method for measuring skin characteristics according to an embodiment of the invention. The method can be performed by apparatus 100 or 200 described above, according to either the embodiment of Fig. 4 or Fig. 5.

Step 601 comprises receiving, over time period T, the first (detector) response indicating reflected intensities (from skin) of light of a first wavelength $\lambda_1$. The intensities may be modulated at a frequency f1. Step 601 further comprises receiving, over the same period T, a second (detector) response which indicates reflected intensities (from skin) of light of a second wavelength $\lambda_2$. The intensities may be modulated at a frequency f2 - f1 and f2 may be the same frequency, or different frequencies.

Step 602 comprises receiving a first indication and second indication, corresponding to total intensities of light emitted over time period T by each light source 201 and 202, respectively, towards the skin (the incident light), i.e. the intensities I_201_1, I_201_2, ...., I_201_i, i> 1 and I_202_1, I_202_2, ...., I_202_i, i> 1 emitted at the first wavelength $\lambda_1$, and the second wavelength $\lambda_2$, respectively. The order of steps 601 and 602 is immaterial to the working of the method (or apparatus). As mentioned above, the term "total" herein is intended to emphasize the original emitted intensities without optical path losses, and in principle, is the emitted intensity of each source during T.

The intensities may be transmitted in real time, or after period T.

In step 603, a variation 10, 11, is obtained, or a response curve is traced, with respect to each of the received first (detector) response vs indication of total intensity emitted by source 201 and the received second (detector) response vs indication of total intensity emitted by source 202. Step 604 comprises identifying a substantially linear portion 10', 11', in each obtained variation 10, 11, where the detector response varies linearly with the emitted intensity of either light source. It is possible to combine these steps, or omit step 603, and thus directly identifying the linear regime of the detector.

The method further comprises, in step 605, obtaining the respective slope at at least a point of the substantially linear portion, i.e. in the linear regime of the detector. The skin characteristic is then calculated in step 606 as a function of the ratio of slopes. In other words, the ratio of the slopes is mapped to the skin characteristic.

As mentioned above, in an embodiment, light sources 201 and 202 are configured to emit intensities over a range of intensity values.

In another embodiment, the light source 201 and/or 202 are configured to emit intensities at selected values (e.g. 2 different intensities $I_m$ and $I_n$ ) which lie in the linear regime of detector 203.

In this case, the processor 1001 or 2001 is configured to receive the respective first response and the first total intensity comprising reflected intensities $I_{m'}$ and $I_{n'}$ emitted intensities $I_m$ and $I_n$ at the first wavelength $\lambda_1$ (i.e. corresponding to source 201), respectively, and the second response and the second total intensity comprising reflected and emitted intensities at the second wavelength, respectively, and/or their indications, calculate the skin characteristic based on a ratio of slopes obtained as

$$\frac{[\frac{In'-Im'}{In-Im}]\lambda_1}{[\frac{In'-Im'}{In-Im}]\lambda_2} \text{, alternately, } \frac{[\frac{In'-Im'}{In-Im}]_{201}}{[\frac{In'-Im'}{In-Im}]_{202}} \qquad\qquad \text{Equation 1}$$

Alternately or in addition, while using two or more selected intensities, the processor 1001 or 2001 is configured to calculate a reflection ratio S based on a ratio of a difference of light intensities at the first wavelength to a difference of light intensities at the second wavelength, wherein the light intensities e.g. In and Im, are emitted at any two instants in the time period T and associated with the linear portion of the response curve.
Specifically, the reflection ratio S is calculated as

$$\frac{[\eta_{PD}\eta_{skin}R_{skin}(I_n-I_m)]_{\lambda_1}}{[\eta_{PD}\eta_{skin}R_{skin}(I_n-I_m)]_{\lambda_2}}, \qquad\qquad \text{Equation 2}$$

- $\eta_{skin}$ is a ratio of light of a certain wavelength which is reflected from the skin and incident on detector 203 to the total emitted light intensity
- $R_{skin}$ is a skin reflection coefficient at the wavelength
- $I_m$ and $I_n$ are values of the total light intensities which are emitted over the time period T and associated with the linear portion of the response curve, and $I_n < I_n$
- $\eta_{PD}$ is a measure of efficiency of detector 203, which is the ability to convert the light incident on detector 203 into an electric signal (response - current or voltage) at the respective wavelength .

The ratio can also be calculated based on the respective responses received by the processor (equation 10), which are a function of the above emitted intensities.

It then maps the reflection ratio S to the skin characteristic.

As mentioned, it is yet another object of the invention to provide a signal measurement method which compensates for reflection losses because of internal reflections in device or the optical sensor therein.

Not all light that is emitted by the light sources 201 and 202 will reach the skin. Some of the light (total emitted intensity) will be scattered on air-light guide interfaces, light guide imperfections, or (other) components inside the device 200. A part of this scattered light will also reach detector 203 and be detected by detector 203 together with the light that is (back)scattered or reflected by the skin. This light that is generated by either light source 201, 202 and detected by the detector 203, without having interacted with the skin, is regarded as "internal reflection" for the purpose of this invention.

The device (processor 1001 or 2001) and the method thereof calculates the skin characteristic which is corrected based on an intensity of a part of the total emitted light reflected internally within the optical sensor at each wavelength. The intensity of the internally reflected light is proportional to a difference of light intensities $I_m$ and $I_n$ which are emitted at any two distinct instants in the time period T (or to detector responses e.g. voltages obtained for these intensities) and associated with the linear portion of the curve. This further improves the skin characteristic calculated as per the embodiment of Fig. 6.

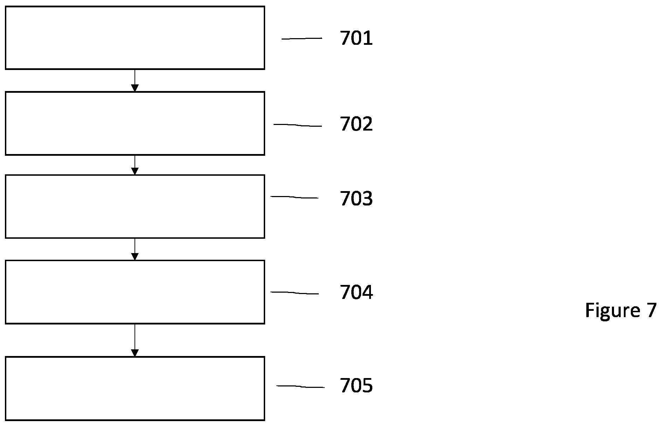

Fig. 7 shows a method according to an embodiment of the invention, detailing the above aspect of the invention, and can be performed by the apparatus 100 or 200 described in the above embodiments.

According to an embodiment, a method to reduce noise from internal reflection comprises in a first step 701, measuring a reflectance signal detected by detector 203, with light sources 201 and 202 emitting light of wavelengths $\lambda_1$, $\lambda_2$ switched on at intensity I_n_201, I_n_202, respectively. Both intensities lie in the linear portion 10', 11' of the response curves 10, 11, i.e. they have values higher than 1_0_201 and I_0_202, respectively. The measured reflectance signals are denoted as Sg1_201 and Sg1_202. The signals can be measured simultaneously or sequentially.

Measurement signal Sg1 comprises contributions from the internal reflection, reflection of light from the skin and ambient light, as a function of the emitted intensities from light sources 201, 202. As mentioned above, at emitted intensity threshold I_0 of respective light source 201, 202, the detector response transitions from a non-linear to a linear regime. The contribution from internal reflection includes intensities which lie both below and above I_0. The reflection of ambient broadband (bb) light is also included. $\eta_{PD}^{bb}$ is a measure of efficiency of detector 203 to convert broadband ambient light incident thereupon into an electric signal (response - current or voltage).

Signal Sg1 can be expressed as:

$$Sg1\_201=$$

$$\eta_{PD\lambda_1}^{nl} \cdot \eta_{IntRefl\lambda_1} \cdot I_{0\_201} + \eta_{PD\lambda_1} \cdot \eta_{IntRefl\lambda_1} \cdot \left(I_{n\_201} - I_{0\_201}\right) + \eta_{PD\lambda_1} \cdot \eta_{skin\lambda_1} \cdot R_{skin} \cdot I_{n\_201} + \eta_{PD}^{bb}$$
$$\cdot \eta_{amb} \cdot I_{amb}$$

Equation 3

$$Sg1\_202=$$

$$\eta_{PD\lambda_2}^{nl} \cdot \eta_{IntRefl\lambda_2} \cdot I_{0\_202} + \eta_{PD\lambda_2} \cdot \eta_{IntRefl\lambda_2} \cdot \left(I_{n\_202} - I_{0\_202}\right) + \eta_{PD\lambda_2} \cdot \eta_{skin\lambda_2} \cdot R_{skin} \cdot I_{n\_202} + \eta_{PD}^{bb}$$
$$\cdot \eta_{amb} \cdot I_{amb}$$

Equation 4

In a second step 702, the method comprises measuring a reflectance signal detected by detector 203, again with light source 201 and 202 switched on. The emitted intensity of each light source in this case is however only sufficiently high to lie in the linear portion 10'/11' above I_0_201 and I_0_202, and have values lower than I_n_201 and 1_n_202. These intensities are denoted as I_m_201 and I_m_202, respectively. The measured signals are denoted as Sg2_201 and Sg2_202, and as above, a function of the emitted intensities from light sources 201, 202.

Signal Sg2 can be expressed as:

$$Sg2\_201=$$

$$\eta_{PD\lambda_1}^{nl} \cdot \eta_{IntRefl\lambda_1} \cdot I_{0\_201} + \eta_{PD\lambda_1} \cdot \eta_{IntRefl\lambda_1} \cdot \left(I_{m\_201} - I_{0\_201}\right) + \eta_{PD\lambda_1} \cdot \eta_{skin\lambda_1} \cdot R_{skin} \cdot I_{m\_201} + \eta_{PD}^{bb}$$
$$\cdot \eta_{amb} \cdot I_{amb}$$

Equation 5

$$Sg2\_202=$$

$$\eta_{PD\lambda_2}^{nl} \cdot \eta_{IntRefl\lambda_2} \cdot I_{0\_202} + \eta_{PD\lambda_2} \cdot \eta_{IntRefl\lambda_2} \cdot \left(I_{m\_202} - I_{0\_202}\right) + \eta_{PD\lambda_2} \cdot \eta_{skin\lambda_2} \cdot R_{skin} \cdot I_{m\_202} + \eta_{PD}^{bb}$$
$$\cdot \eta_{amb} \cdot I_{amb}$$

Equation 6

Measurement signal Sg2 comprises contributions from the internal reflection, reflection of light from the skin and ambient light.

Therefore, both signals Sg1 and Sg2 include contributions from internal reflections in the device. A part of this contribution lies in the non-linear regime of the response curves 10, 11, and another part, in the linear regime expressed by portions 10' and 11', respectively. The former is indicated by superscript *nl* in equations 3-6.

In a third step 703, signals Sg1 and Sg2 are processed for each light source 201 and 202, to obtain a difference signal, Sg1-Sg2 as:

$$(\text{Sg1-Sg2})\_201=$$
$$\eta_{PD\lambda_1} \cdot \eta_{skin\lambda_1} \cdot R_{skin} \cdot \left(I_{n\_201} - I_{m\_201}\right) + \eta_{PD\lambda_1} \cdot \eta_{IntRefl\lambda_1} \cdot \left(I_{n\_201} - I_{m\_201}\right)$$

Equation 7

$$(\text{Sg1-Sg2})\_202=$$
$$\eta_{PD\lambda_2} \cdot \eta_{skin\lambda_2} \cdot R_{skin} \cdot \left(I_{n\_202} - I_{m\_202}\right) + \eta_{PD\lambda_2} \cdot \eta_{IntRefl\lambda_2} \cdot \left(I_{n\_202} - I_{m\_202}\right)$$

Equation 8

Here,

- $\eta_{IntRefl}$ is a ratio of internally reflected light within the optical sensor which is incident on detector 203 to the total emitted light intensity,
- $I_{amb}$ = intensity of the ambient light
- $\eta_{amb}$ is a ratio of ambient light which is reflected from the skin and detected by detector 203 to the total amount of (or incident) ambient light. The remaining terms are as described above.

As seen above, although the contribution of internal reflection in the non-linear response range of detector 203 and the ambient light cancel out, the contribution from the internal reflection that is in the linear response range does not. $\eta_{PD} \cdot \eta_{IntRefl} \cdot (I_n - I_m)$ represents the reflected signal which arises due to scattering from the internally reflected light within the device 200. It is desirable to remove this contribution from the detected reflectance signal.

Steps 701-703 may also be carried out as a calibration step while manufacturing apparatus 100 or 200, i.e. as part of a manufacturing method. The magnitudes of $\eta_{PD} \cdot \eta_{IntRefl} \cdot (I_{n\_201} - I_{m\_201})$ and $\eta_{PD} \cdot \eta_{IntRefl} \cdot (I_{n\_202} - I_{m\_202})$ are stored as calibration data of device 200 and can be used by processor 1001 or 2001, as mentioned above. They can further be stored in the memory 1001 or 2001.

In step 704, processor 1001 or 2001 is configured to retrieve the stored magnitudes during calculation of the skin characteristic and/or receive emitted intensities $I_{n\_201}$, $I_{m\_201}$ and $I_{n\_202}$, $I_{m\_202}$, n>m, relating to light source 201 and light source 202, respectively from device 200, as calibration data to calculate the internal reflection contribution. $I_{m\_201}$ and $I_{m\_202}$ may be equal to or near the thresholds $I_{0\_201}$ and $I_{0\_202}$.

In step 705, once each difference signal (Sg1-Sg2)_201 ( or S1) and (Sg1-Sg2)_202(or S2) are corrected for internal reflection in device 200, the processor is configured to calculate the reflection ratio

$$S \text{ as } \frac{[\eta_{PD}\eta_{skin}R_{skin}(I_n - I_m)]_{\lambda_1}}{[\eta_{PD}\eta_{skin}R_{skin}(I_n - I_m)]_{\lambda_2}} \text{ or } \frac{[\eta_{PD} \cdot \eta_{skin} \cdot R_{skin}]_{\lambda_1} \cdot (I_{n\_201} - I_{m\_201})}{[\eta_{PD} \cdot \eta_{skin} \cdot R_{skin}]_{\lambda_2} \cdot (I_{n\_202} - I_{m\_202})}$$

Equation 9

The above reflection ratio S essentially corresponds to $\frac{[(Sg1-Sg2) - contribution\ from\ internal\ reflection]_{\lambda_1}}{[(Sg1-Sg2) - contribution\ from\ internal\ reflection]_{\lambda_2}}$. In other words, the processor 1001 or 2001 adjusts the response obtained at each wavelength based on a measure of internal reflection inside the optical sensor.

In step 705, the reflection ratio S calculated using the ratio of slopes as described in Figs. 4-6 is alternately or in addition adjusted using the calibration data obtained by the processor in step 704.

As mentioned with reference to Fig. 3 and throughout the above description, instead of working directly with emitted and reflected intensities, the method and the apparatus of the present invention may use the driving voltage or current values of light sources 201 and 202, and correspondingly, the voltage or current generated by detector 203 as the first and the second response. Since the magnitude for internal reflection is a function of the detector conversion efficiency (equation 9), the calibration data may also be expressed in terms of the detector responses measured for the emitted intensities $I_{n\_201}$, $I_{m\_201}$ and $I_{n\_202}$, $I_{m\_202}$. In this case, the reflection ratio S may be approximated in terms of the first response (corresponding to source 201) and the second response (corresponding to source 202) of detector 203, for example, as

$$\frac{\left[V_{PS,V_{201\_n}} - V_{PS,V_{201\_m}}\right]_{sensor\ positioned\ on\ skin} - \left[V_{PS,V_{201,n}} - V_{PS,V_{201,m}}\right]_{int\ ref}}{\left[V_{PS,V_{202\_n}} - V_{PS,V_{202\_m}}\right]_{sensor\ positioned\ on\ skin} - \left[V_{PS,V_{202,n}} - V_{PS,V_{202,m}}\right]_{int\ ref}} \qquad \text{Equation 10}$$

Here, $[V_{PS,V_{201,n}} - V_{PS,V_{201,m}}]_{int\ ref}$ corresponds to a reference response received by processor 1001 or 2001. It is a difference of voltage responses obtained at different driving voltages of light source 201 and when device 200 (optical sensor) is positioned facing an optical black box during a calibration step [as for above steps 701-703]. This can be stored in memory 1002 or 2002 and retrieved by processor 1001 or 2001 for adjusting the first response.

$[V_{PS,V_{202,n}} - V_{PS,V_{202,m}}]_{int\ ref}$ corresponds to a second reference response received by processor 1001 or 2001. It is obtained in a manner similar to the first reference response, with light source 202.

Equation 9 and 10 are similar, and therefore equation 10 can be used interchangeably with equation 9 together with the respective stored or measured voltage calibration data, to remove contribution of internal reflection from the detected response.

To obtain the above voltage responses and their difference, the driving voltages to the light sources 201 and 202 are chosen such that responses $(V_{PS,V_{201,n}}, V_{PS,V_{201,m}})$ and $(V_{PS,V_{202,n}}, V_{PS,V_{202,m}})$ both lie in the linear portion of the curve.

Thus, the processor adjusts the first response and the second response based on an indicator or parameter (the intensity, voltage, current,..) which represents a part of the total emitted light reflected internally within the optical sensor, wherein said indicator is determined based on the linear portion of the respective variation curve.

In an embodiment, device 200 further comprises a temperature sensor (not shown) to measure temperature of circuitry which control the operation of at least each of the light source 201, 202, and detector 203. Processor 1001 or 2001 is configured to adjust the first response and/or the second response based on the temperature measurements of the sensor.

Processor 1001 or 2001 may be configured to receive measurements of the temperature sensor during the period T, and further, to offset the measured temperature using a reference temperature. This step is performed to compensate for temperature of the surrounding environment and to compensate for thermal influences on device 100 or 200. The reflection ratio S, and hence the skin characteristic, are then calculated based on compensated temperature and hence temperature compensated light signals.

The reference temperature for performing temperature correction can be stored as calibration data in memory 1002 or 2002.

According to the methods of the above-mentioned embodiments, once the reflectance ratio S is obtained, the ratio can be mapped to the skin characteristic using suitable mathematic functions.

In an embodiment, the determined skin characteristic is a skin tone. In another embodiment, it is skin color or a degree of redness. A plurality of skin characteristics may also be determined by device 100 or 200, depending on the wavelengths of the light source used.

In yet another embodiment, a personal care device which comprises the apparatus described above, or which apparatus is suitable to perform any of the methods described above, is provided.

When apparatus 200 is an optical sensor, such sensor may be included in an attachment of the personal care device. This attachment may be calibrated in a factory setting as mentioned in any of the above embodiments and made available for use by the user in the client device 200.

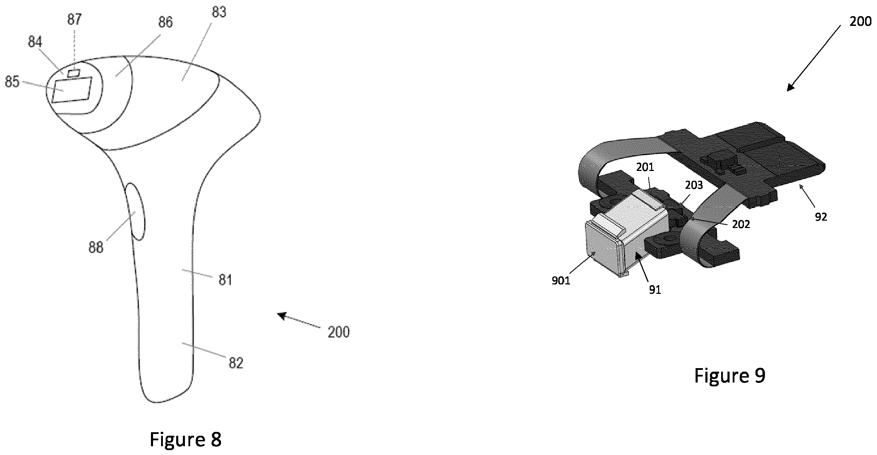

Fig. 8 shows an illustration of an exemplary personal care device 200 that can be used to apply light to skin for purposes of skin treatment. It will be appreciated that the device 200 in the figure is merely presented as an example of device 200 that the invention can be used with, and the device 200 is not limited to the form shown in Fig. 8 or to being a handheld treatment device. It may comprise additional components than those described below in connection with the figure. As noted above, the invention is likewise not limited to being implemented in or with device 200, and in some embodiments the invention can be implemented in a remote server, or in another client apparatus that can be provided for the purpose of determining skin characteristics.

The device 200 is for use on a body of a subject (e.g. a person or an animal) and is to be held by a user during use. Device 200 is to perform some treatment operation to hairs on the body of the subject using one or more light pulses when it is in contact with a body part of the subject.

The exemplary personal care device 200 comprises a housing 81 that includes at least a handle portion 82 and a head portion 83. The handle portion 82 is shaped to enable the user to hold device 200 with one hand. The head portion 83 has a head end 84 that is to be placed into contact with the subject in order for the treatment operation to be performed on the body or skin of the subject at the position that the head end 84 is in contact with the body or skin.

The device 200 is for performing a treatment operation using light pulses. Thus, in Fig. 8, the head end 84 comprises an aperture 85 that is arranged in or on the housing 81 so that the aperture 85 can be placed adjacent to or on (i.e. in contact with) the skin of the subject. The personal care device 200 includes one or more light sources 86, e.g. inside the head portion 83, that are configured for generating light pulses to be applied to the skin of the subject via the aperture 85 and effect a treatment operation. The one or more light sources 86 are arranged in the housing 81 so that the light pulses are provided from the one or more light sources 86 through the aperture 85.

The one or more light sources 86 can generate light pulses of any suitable or desired wavelength (or range of wavelengths) and/or intensities. The one or more light sources 86 are configured to provide pulses of light. In case of a single light source 86, a plurality of optical filters may be used to separate the respective wavelength components. That is, the light source(s) 86 are configured to generate light at a high intensity for a short duration (e.g. less than 1 second). The intensity of the light pulse should be high enough to effect the treatment operation on the skin or body part adjacent the aperture 85. For example, the light sources 86 can generate visible light, infra-red (IR) light and/or ultraviolet (UV) light. Each light source 86 can comprise any suitable type of light source, such as one or more light emitting diodes (LEDs), a (Xenon) flash lamp, a laser or lasers, etc. The light sources 86 can provide light pulses with spectral content in the 530-1200 nanometre (nm) range.

In addition to the one or more light sources 86 for effecting the light-based treatment operation, the device also comprises an apparatus (sensor 87) for determining a value of a skin characteristic, such as skin tone. Sensor 87 comprises one or more light source(s) and one or more light sensor(s). In Fig. 8, the sensor 87 is shown disposed above aperture 85, however, sensor 87 may be located at a different position relative to the aperture 85 on device 200. In case of multiple sensors 87, these may be positioned on either opposite side of aperture 85. As mentioned above, the determination of the skin characteristic can be made by device 200 or the sensor 87 therein. It can also be determined by an apparatus 100 distributed from device 200. The above embodiments detail the use of two light sources 201 and 202, but it is understood that a skin reflectance and hence the skin characteristic, can be obtained using intensities of a single light source. In this case, the further step of obtaining ratio of slopes is omitted.

The device 200 also includes a user control 88 that can be operated by the user to activate the personal care device so that the required treatment operation is performed on the body of the subject (e.g. the generation of one or more light pulses by the one or more light source(s) 86). The user control 88 may be in the form of a switch, a button, a touch pad, etc.

Light sources 86, sensor 87 and user control 88 may be connected to processor 2001 (not shown in Fig. 8 but in Fig. 5), the latter can be implemented in numerous ways, with software and/or hardware, to perform the various functions described herein. For example, processor 2001 may comprise one or more microprocessors or digital signal processors (DSPs) that may be programmed using software or computer program code to perform the required functions and/or to control components of the processor 2001 to effect the required functions. The processor 2001 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), field-programmable gate arrays (FPGAs), hardware for implementing a neural network and/or so-called artificial intelligence (AI) hardware accelerators (i.e. a processor(s) or other hardware specifically designed for AI applications that can be used alongside a main processor).

As mentioned, the processor 2001 can comprise or be associated with a memory 2002. The memory 2002 can store data, information and/or signals (including image(s)) for use by the processor 2001 in controlling the operation of the device 200 and/or in executing or performing the methods described herein. In some implementations the memory 2002 stores computer-readable code that can be executed by the processor 2001 so that the processor 2001 performs one or more functions, including the methods described herein. In particular embodiments, the program code can be in the form of an application for a smart phone, tablet, laptop, computer or server. The memory 2002 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM) and electrically erasable PROM (EEPROM), and the memory unit can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc. The details of processor 2001 and memory 2002 apply equally to processor 1001 and 1002 of apparatus 100, which carries out the invention according to the above embodiments.

At least when the invention is carried out in another apparatus 100, a transceiver 207 of device 200 enables a data connection to and/or data exchange with other devices 100, including any one or more of servers, databases, user devices, and sensors. It can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). It may further comprise circuitry to control any suitable input component(s), including but not limited to a keyboard, keypad, one or more buttons, switches or dials, a mouse, a track pad, a touchscreen, a stylus, a camera, a microphone, etc., and the user interface can comprise any suitable output component(s), including but not limited to a display unit or display screen, one or more lights or light elements, one or more loudspeakers, a vibrating element, etc.

In an embodiment as shown in Fig. 9, sensor 87 comprises light sources 201 and 202 disposed on opposite ends of a main body 91. As shown in the figure, the light sources 201 and 202 may be implemented as LED strips. The sources may alternately take any other suitable form. Detector 203 is positioned in a spacing between the light sources 201 and 202 and behind the main body 91. The main body 91 functions as a light guide to transport reflected light intensities towards detector 203. A head 901 of the main body 91 distal from the detector 203 may be in contact with skin when device 200 is in use. The main body 91 may further include additional elements, e.g. those mentioned in the embodiments of Figs. 4 or 5, temperature sensor(s), the processing circuitry, and the like. The processing circuitry may be alternately or in addition disposed in the main circuit board 92 connected to the sources 201, 202, detector 203 and the main body 91.

**Claims**

1. An apparatus (100, 200) for measuring a skin characteristic, the apparatus comprising:

   a processor (1001, 2001) configured to

   - receive, over a time period T, a first response indicating intensities of light of a first wavelength $\lambda_1$ reflected from skin, and

   a second response indicating intensities of light of a second wavelength $\lambda_2$ reflected from the skin;

   - receive a first indication of a total intensity of light corresponding to the first response and a second indication of a total intensity of light corresponding to the second response, which are emitted to the skin over the time period T;
   - determine a first variation curve (10) with the first response as a function of the first indication and a second variation curve (11) with the second response as a function of the second indication;
   - determine a portion (10', 11') of each variation curve, along which portion (10', 11') the variation is substantially linear;
   - calculate slopes at at least one point in said portion of the variation curves, and
   - determine a value of the skin characteristic from a ratio of the slopes.

2. The apparatus (200) of claim 1 further comprising:

   a first light source (201) and a second light source (202) configured to emit the first total intensity of light of the first wavelength $\lambda_1$ and the second total intensity of light of the second wavelength $\lambda_2$, respectively, to the skin; a detector (203) configured to detect the first response and the second response, and/or a signal generator (204) configured to modulate an intensity of the emitted light by the first light source (201) at a first frequency f1 and the second light source (202) at a second frequency f2 across a plurality of intensities during the time period T.

3. The apparatus of claim 2, comprising at least one further detector (205, 206) configured to detect an intensity of the emitted light from the first light source (201) or the second light source (202).

4. The apparatus of any of claims 2 - 3, wherein the signal generator (204) is further configured to modulate the intensity simultaneously or sequentially, and/or the first frequency and the second frequency are same or different.

5. The apparatus of any of claims 1-4, wherein the indication and response include voltage, current or intensity parameters.

6. The apparatus of any of claims 1-5, wherein the first and/or the second wavelength is a visible or an infrared wavelength.

7. The apparatus of any of claims 1-6 being an optical sensor, and wherein the processor (1001, 2001) is further configured to adjust the first response and the second response based on an intensity of a part of the total emitted light reflected internally within the optical sensor, wherein the intensity of said part is determined based on total light intensities emitted over the time period T and associated with said portion of the variation curve.

8. The apparatus of any of claims 1-7, further comprising a temperature sensor, and wherein the processor (1001, 2001) is further configured to adjust the first response and/or the second response based on a measurement of the temperature sensor.

9. The apparatus of any of claims 1-8, wherein the processor (1001, 2001) is further configured to determine a reflection ratio S based on a ratio of a difference of light intensities at the first wavelength to a difference of light intensities at the second wavelength, wherein the light intensities are emitted at any two instants in the time period T and associated with said portion of the variation curve, and to map the reflection ratio to the skin characteristic.

10. The apparatus of any of claims 1-9, further comprising a memory (1002, 2002) to store a calibration correction for the first response and/or the second response, and wherein the processor (1001, 2001) is further configured to determine the value of the skin characteristic based on the calibration correction.

11. The apparatus of any of claims 1-10, wherein the skin characteristic is a skin tone or skin pigmentation/color.

12. A personal care device comprising the apparatus of any of claims 1-11, and/or wherein the apparatus is included in an attachment of the personal care device.

13. A computer-implemented method for determining a skin characteristic, the method comprising:

receiving (301), over a time period T, a first response indicating intensities of light of a first wavelength $\lambda_1$ reflected from skin, and
a second response indicating intensities of light of a second wavelength $\lambda_2$ reflected from the skin;

- receiving (302) a first indication of a total intensity of light corresponding to the first response and a second indication of a total intensity of light corresponding to the second response, which are emitted to the skin over the time period T;
- determining (303) a first variation curve (10) with the first response as a function of the first indication and a second variation curve (11) with the second response as a function of the second indication;
- determining (304) a portion (10', 11') of each variation curve, along which portion (10', 11') the variation is substantially linear;
- calculating (305) slopes at at least one point in said portion of the variation curves, and
- determining (306) a value of the skin characteristic from a ratio of the slopes.

14. The method of claim 13, further comprising steps carried out by the apparatus of any of claims 1-11.

15. A computer program product or a computer-readable storage medium comprising instructions which, when the program is executed by a computer, cause the computer to carry out the steps of the method of claims 13 and/or 14.

**Patentansprüche**

1. Einrichtung (100, 200) zum Messen einer Hauteigenschaft, wobei die Einrichtung umfasst:

einen Prozessor (1001, 2001), der konfiguriert ist, um

- über einen Zeitraum T eine erste Antwort zu empfangen, die Intensitäten von Licht einer ersten Wellenlänge $\lambda_1$, das von der Haut reflektiert wird, angibt, und

eine zweite Antwort, die Intensitäten von Licht einer zweiten Wellenlänge $\lambda_2$, das von der Haut reflektiert wird, angibt;

- eine erste Angabe einer Gesamtintensität von Licht, die der ersten Antwort entspricht, und eine zweite Angabe einer Gesamtintensität von Licht, die der zweiten Antwort entspricht, die über den Zeitraum T auf die Haut emittiert werden, zu empfangen;
- eine erste Variationskurve (10) mit der ersten Antwort in Abhängigkeit von der ersten Angabe und eine zweite Variationskurve (11) mit der zweiten Antwort in Abhängigkeit von der zweiten Angabe zu bestimmen;
- einen Abschnitt (10', 11') jeder Variationskurve zu bestimmen, entlang welchen Abschnitts (10', 11') die Variation im Wesentlichen linear ist;
- Steigungen an mindestens einem Punkt in dem Abschnitt der Variationskurven zu berechnen, und
- aus einem Verhältnis der Steigungen einen Wert der Hauteigenschaft zu bestimmen.

2. Einrichtung (200) nach Anspruch 1, weiter umfassend:

eine erste Lichtquelle (201) und eine zweite Lichtquelle (202), die konfiguriert sind, um jeweils die erste Gesamtintensität von Licht der ersten Wellenlänge $\lambda_1$ und die zweite Gesamtintensität von Licht der zweiten Wellenlänge $\lambda_2$ auf die Haut zu emittieren;
einen Detektor (203), der konfiguriert ist, um die erste Antwort und die zweite Antwort zu detektieren, und/oder
einen Signalgenerator (204), der konfiguriert ist, um eine Intensität des von der ersten Lichtquelle (201) mit einer ersten Frequenz f1 und der zweiten Lichtquelle (202) mit einer zweiten Frequenz f2 emittierten Lichts während des Zeitraums T über eine Vielzahl von Intensitäten zu modulieren.

3. Einrichtung nach Anspruch 2, die mindestens einen weiteren Detektor (205, 206) umfasst, der konfiguriert ist, um eine Intensität des von der ersten Lichtquelle (201) oder der zweiten Lichtquelle (202) emittierten Lichts zu detektieren.

4. Einrichtung nach einem der Ansprüche 2 - 3, wobei der Signalgenerator (204) weiter konfiguriert ist, um die Intensität gleichzeitig oder nacheinander zu modulieren, und/oder die erste Frequenz und die zweite Frequenz gleich oder unterschiedlich sind.

5. Einrichtung nach einem der Ansprüche 1-4, wobei die Angabe und Antwort Spannungs-, Strom- oder Intensitätsparameter beinhalten.

6. Einrichtung nach einem der Ansprüche 1-5, wobei die erste und/oder die zweite Wellenlänge eine sichtbare oder eine Infrarot-Wellenlänge sind/ist.

7. Einrichtung nach einem der Ansprüche 1-6, die ein optischer Sensor ist, und wobei der Prozessor (1001, 2001) weiter konfiguriert ist, um die erste Antwort und die zweite Antwort auf Basis einer Intensität eines Teils des gesamten emittierten Lichts, das intern innerhalb des optischen Sensors reflektiert wird, anzupassen, wobei die Intensität des Teils auf Basis von Gesamtlichtintensitäten, die über den Zeitraum T emittiert werden und dem Abschnitt der Variationskurve zugeordnet sind, bestimmt wird.

8. Einrichtung nach einem der Ansprüche 1-7, die weiter einen Temperatursensor umfasst, und wobei der Prozessor (1001, 2001) weiter konfiguriert ist, um die erste Antwort und/oder die zweite Antwort auf Basis einer Messung des Temperatursensors anzupassen.

9. Einrichtung nach einem der Ansprüche 1-8, wobei der Prozessor (1001, 2001) weiter konfiguriert ist, um ein Reflexionsverhältnis S auf Basis eines Verhältnisses einer Differenz von Lichtintensitäten bei der ersten Wellenlänge zu einer Differenz von Lichtintensitäten bei der zweiten Wellenlänge zu bestimmen, wobei die Lichtintensitäten zu zwei beliebigen Zeitpunkten im Zeitraum T emittiert werden und dem Abschnitt der Variationskurve zugeordnet sind, und um das Reflexionsverhältnis auf die Hauteigenschaft abzubilden.

10. Einrichtung nach einem der Ansprüche 1-9, die weiter einen Speicher (1002, 2002) umfasst, um eine Kalibrierungskorrektur für die erste Antwort und/oder die zweite Antwort zu speichern, und wobei der Prozessor (1001, 2001) weiter konfiguriert ist, um den Wert der Hauteigenschaft auf Basis der Kalibrierungskorrektur zu bestimmen.

11. Einrichtung nach einem der Ansprüche 1-10, wobei die Hauteigenschaft ein Hautton oder eine Hautpigmentierung/-farbe ist.

12. Körperpflegevorrichtung, umfassend die Einrichtung nach einem der Ansprüche 1-11, und/oder wobei die Einrichtung in einem Aufsatz der Körperpflegevorrichtung beinhaltet ist.

13. Computerimplementiertes Verfahren zum Bestimmen einer Hauteigenschaft, wobei das Verfahren umfasst:

Empfangen (301) über einen Zeitraum T einer ersten Antwort, die Intensitäten von Licht einer ersten Wellenlänge $\lambda_1$, das von der Haut reflektiert wird, angibt, und einer zweiten Antwort, die Intensitäten von Licht einer zweiten Wellenlänge $\lambda_2$, das von der Haut reflektiert wird, angibt;

- Empfangen (302) einer ersten Angabe einer Gesamtintensität von Licht, die der ersten Antwort entspricht, und eine zweite Angabe einer Gesamtintensität von Licht, die der zweiten Antwort entspricht, die über den Zeitraum T auf die Haut emittiert werden;
- Bestimmen (303) einer ersten Variationskurve (10) mit der ersten Antwort in Abhängigkeit von der ersten Angabe und eine zweite Variationskurve (11) mit der zweiten Antwort in Abhängigkeit von der zweiten Angabe;
- Bestimmen (304) eines Abschnitts (10', 11') jeder Variationskurve, entlang welchen Abschnitts (10', 11') die Variation im Wesentlichen linear ist;
- Berechnen (305) von Steigungen an mindestens einem Punkt in dem Abschnitt der Variationskurven, und
- Bestimmen (306) eines Wertes der Hauteigenschaft anhand eines Verhältnisses der Steigungen.

14. Verfahren nach Anspruch 13, das weiter Schritte umfasst, die von der Einrichtung nach einem der Ansprüche 1-11 ausgeführt werden.

15. Computerprogrammprodukt oder computerlesbares Speichermedium, das Anweisungen umfasst, die, wenn das Programm von einem Computer ausgeführt wird, den Computer veranlassen, die Schritte des Verfahrens nach den Ansprüchen 13 und/oder 14 auszuführen.

**Revendications**

1. Appareil (100, 200) pour mesurer une caractéristique de la peau, l'appareil comprenant :

un processeur (1001, 2001) configuré pour

- recevoir, sur une période de temps T, une première réponse indiquant des intensités de lumière d'une première longueur d'onde $\lambda_1$ réfléchie par la peau, et

une seconde réponse indiquant des intensités de lumière d'une seconde longueur d'onde $\lambda_2$ réfléchie par la peau ;

- recevoir une première indication d'une intensité totale de lumière correspondant à la première réponse et une seconde indication d'une intensité totale de lumière correspondant à la seconde réponse, qui sont émises vers la peau sur la période de temps T;
- déterminer une première courbe de variation (10) avec la première réponse en fonction de la première indication et une seconde courbe de variation (11) avec la seconde réponse en fonction de la seconde indication ;
- déterminer une portion (10', 11') de chaque courbe de variation, le long de laquelle portion (10', 11') la variation est sensiblement linéaire ;
- calculer les pentes en au moins un point de ladite portion des courbes de variation, et
- déterminer une valeur de la caractéristique de la peau à partir d'un rapport des pentes.

2. Appareil (200) selon la revendication 1, comprenant en outre :

une première source de lumière (201) et une seconde source de lumière (202) configurées pour émettre la première intensité totale de lumière de la première longueur d'onde $\lambda_1$ et la seconde intensité totale de lumière de la seconde longueur d'onde $\lambda_2$, respectivement, vers la peau ; un détecteur (203) configuré pour détecter la première réponse et la seconde réponse, et/ou

un générateur de signal (204) configuré pour moduler une intensité de la lumière émise par la première source de lumière (201) à une première fréquence f1 et la seconde source de lumière (202) à une seconde fréquence f2 sur une pluralité d'intensités pendant la période de temps T.

3. Appareil selon la revendication 2, comprenant au moins un autre détecteur (205, 206) configuré pour détecter une intensité de la lumière émise par la première source de lumière (201) ou la seconde source de lumière (202).

4. Appareil selon l'une quelconque des revendications 2-3, dans lequel le générateur de signal (204) est en outre configuré pour moduler l'intensité simultanément ou séquentiellement, et/ou la première fréquence et la seconde fréquence sont identiques ou différentes.

5. Appareil selon l'une quelconque des revendications 1-4, dans lequel l'indication et la réponse incluent des paramètres de tension, de courant ou d'intensité.

6. Appareil selon l'une quelconque des revendications 1-5, dans lequel la première et/ou la seconde longueur d'onde est une longueur d'onde visible ou infrarouge.

7. Appareil selon l'une quelconque des revendications 1-6, étant un capteur optique, et dans lequel le processeur (1001, 2001) est en outre configuré pour ajuster la première réponse et la seconde réponse sur la base d'une intensité d'une partie de la lumière totale émise réfléchie en interne dans le capteur optique, dans lequel l'intensité de ladite partie est déterminée sur la base des intensités lumineuses totales émises sur la période de temps T et associées à ladite portion de la courbe de variation.

8. Appareil selon l'une quelconque des revendications 1-7, comprenant en outre un capteur de température, et dans lequel le processeur (1001, 2001) est en outre configuré pour ajuster la première réponse et/ou la seconde réponse basée sur une mesure du capteur de température.

9. Appareil selon l'une quelconque des revendications 1-8, dans lequel le processeur (1001, 2001) est en outre configuré pour déterminer un rapport de réflexion S sur la base d'un rapport d'une différence d'intensités lumineuses à la première longueur d'onde à une différence d'intensités lumineuses à la seconde longueur d'onde, dans lequel les intensités lumineuses sont émises à deux instants quelconques dans la période de temps T et associées à ladite portion de la courbe de variation, et pour mapper le rapport de réflexion à la caractéristique de la peau.

10. Appareil selon l'une quelconque des revendications 1-9, comprenant en outre une mémoire (1002, 2002) pour stocker une correction d'étalonnage pour la première réponse et/ou la seconde réponse, et dans lequel le processeur (1001, 2001) est en outre configuré pour déterminer la valeur de la caractéristique de la peau sur la base de la correction d'étalonnage.

11. Appareil selon l'une quelconque des revendications 1-10, dans lequel la caractéristique de la peau est un teint de peau ou une pigmentation/couleur de peau.

12. Dispositif de soins personnels comprenant l'appareil selon l'une quelconque des revendications 1-11, et/ou dans lequel l'appareil est inclus dans un accessoire du dispositif de soins personnels.

13. Procédé mis en oeuvre par ordinateur pour déterminer une caractéristique de la peau, le procédé comprenant :

la réception (301), sur une période de temps T, d'une première réponse indiquant des intensités de lumière d'une première longueur d'onde $\lambda_1$ réfléchie par la peau, et
d'une seconde réponse indiquant des intensités de lumière d'une seconde longueur d'onde $\lambda_2$ réfléchie par la peau ;

- la réception (302) d'une première indication d'une intensité totale de lumière correspondant à la première réponse et d'une seconde indication d'une intensité totale de lumière correspondant à la seconde réponse, qui sont émises vers la peau sur la période de temps T ;
- la détermination (303) d'une première courbe de variation (10) avec la première réponse en fonction de la première indication et d'une seconde courbe de variation (11) avec la seconde réponse en fonction de la seconde indication ;
- la détermination (304) d'une portion (10', 11') de chaque courbe de variation, le long de laquelle portion (10', 11') la variation est sensiblement linéaire ;
- le calcul (305) des pentes en au moins un point de ladite portion des courbes de variation, et
- la détermination (306) d'une valeur de la caractéristique de la peau à partir d'un rapport des pentes.

14. Procédé selon la revendication 13, comprenant en outre des étapes réalisées par l'appareil selon l'une quelconque des revendications 1-11.

15. Produit de programme informatique ou support de stockage lisible par ordinateur comprenant des instructions qui, lorsque le programme est exécuté par un ordinateur, amènent l'ordinateur à exécuter les étapes du procédé selon les revendications 13 et/ou 14.

Figure 1

Figure 2b

Figure 2a

EP 4 401 618 B1

Figure 3

Figure 4

Figure 5a

Figure 5b

EP 4 401 618 B1

Figure 6

EP 4 401 618 B1

Figure 7

Figure 8

Figure 9

EP 4 401 618 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 102067260 B1
- KR 20210101896 A